# EUROPÄISCHE PATENTSCHRIFT

(11) **EP 0 648 732 B1**
(45) Veröffentlichungstag und Bekanntmachung des Hinweises auf die Patenterteilung: **29.04.1998**
(21) Anmeldenummer: 94115717.4
(22) Anmeldetag: 06.10.1994
(51) Int. Cl.: C07C 57/04, C07C 51/487

(54) **Verfahren zur Reinigung von Roh-(Meth)acrylsäure**
Process for the purification of crude (meth)acrylic acid
Procédé de purification d'acide (méth)acrylique brut

(30) Priorität: 15.10.1993 DE 4335172
(43) Veröffentlichungstag der Anmeldung: 19.04.1995
(73) Patentinhaber: BASF AKTIENGESELLSCHAFT, 67056 Ludwigshafen (DE)
(72) Erfinder: Dockner, Toni, Dr., D-67149 Meckenheim (DE); Nestler, Gerhard, Dr., D-67061 Ludwigshafen (DE); Herbst, Holger, Dr., D-67227 Frankenthal (DE); Lermer, Helmut, Dr., D-67067 Ludwigshafen (DE); Martan, Hans, Dr., D-67227 Frankenthal (DE); Vogel, Herbert, Dr., D-67069 Ludwigshafen (DE); Exner, Herbert, Dr., D-67165 Waldsee (DE)

(56) Entgegenhaltungen:
- EP-A- 0 468 290
- EP-A- 0 535 489
- DE-A- 1 668 609
- DE-B- 2 207 184

## Beschreibung

Die vorliegende Erfindung betrifft ein Verfahren zum Abtrennen von Aldehyden aus Roh-(Meth)acrylsäure, die nach dem Verfahren der katalytischen Gasphasenoxidation erzeugt wurde, bei dem man die Roh-(Meth)acrylsäure mit einem primären Amin und/oder dessen Salzen versetzt und aus dem Gemisch die (Meth)acrylsäure destillativ abtrennt.

(Meth)acrylsäure wird als verkürzte Schreibweise verwendet und steht für Acrylsäure oder Methacrylsäure. Unter einem primären Amin werden solche Verbindungen verstanden, die wenigstens eine -NH₂-Gruppe aufweisen.

(Meth)acrylsäure, entweder für sich oder in Form ihrer Ester, ist insbesondere zur Herstellung von Polymerisaten für die verschiedensten Anwendungsgebiete, z.B. Verwendung als Klebstoffe, von Bedeutung.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propen, Acrolein, tert.-Butanol, iso-Buten, iso-Butan, iso-Butyraldehyd oder Methacrolein erhältlich. Als Ausgangsverbindungen sind aber auch solche denkbar, aus welchen sich die eigentliche C₄-Ausgangsverbindung während der Gasphasenoxidation erst intermediär bildet. Beispielhaft genannt sei der Methylether des tert.-Butanols. Dabei werden diese Ausgangsgase, in der Regel mit inerten Gasen wie Stickstoff, CO₂, gesättigten Kohlenwasserstoffen und/oder Wasserdampf verdünnt, im Gemisch mit Sauerstoff bei erhöhten Temperaturen sowie gegebenenfalls erhöhtem Druck über übergangsmetallische Mischoxidkatalysatoren geleitet, oxidativ in die (Meth)acrylsäure umgewandelt und durch die Aufnahme in ein geeignetes Absorptionsmittel (z.B. Wasser oder ein Gemisch aus 70 bis 75 Gew.-% Diphenylether und 25 bis 30 Gew.-% Diphenyl) aus dem Produktgasstrom abgetrennt (vgl. z.B. EP-A 297 445 und DE-PS 21 36 396).

Nach Entfernung des Absorptionsmittels (und gegebenenfalls zuvor erfolgter Desorption von eine geringe Absorptionsmittellöslichkeit aufweisenden Verunreinigungen durch Abstreifen, z.B. mit Luft) über extraktive und/oder destillative Trennverfahren (z.B. Entfernung des Absorptionsmittels Wasser durch Destillation, azeotrope Destillation oder extraktive Abtrennung der Säure aus der wäßrigen Lösung und anschließende destillative Entfernung des Extraktionsmittels) wird eine Säure erhalten, die hier als Roh-(Meth)acrylsäure bezeichnet wird.

Aufgrund zahlreicher im Verlauf der katalytischen Gasphasenoxidation erfolgenden Parallel- und Folgereaktionen bildet die Roh-(Meth)acrylsäure kein reines Produkt. Vielmehr enthält sie ein Spektrum verschiedener Verunreinigungen (in der Regel in der Größenordnung von ≤ 2 Gew.-%, vgl. EP-B 169 254), das hauptsächlich aus mit den Ausgangsverbindungen der katalytischen Gasphasenoxidation und mit der resultierenden (Meth)acrylsäure chemisch verwandten Aldehyden besteht. In der Regel enthält Roh-(Meth)acrylsäure daher als Verunreinigungen neben Essig-, Ameisen- und Propionsäure in der Regel Formaldehyd, Acetaldehyd, Acrolein, Methacrolein, Propionaldehyd, n-Butyraldehyd, Benzaldehyd, Furfural und Crotonaldehyd.

Für verschiedene Anwendungen der (Meth)acrylsäure wirken sich die in der Roh-(Meth)acrylsäure enthaltenen Verunreinigungen nachteilig aus (vgl. z.B. DE-AS 22 07 184). So kann z.B. die Induktionszeit von Polymerisationsrektionen, d.h. der Zeitraum zwischen dem Erreichen der Polymerisationstemperatur und dem tatsächlichen Beginn der Polymerisation, nicht reproduzierbar oder der Polymerisationsgrad reduziert sein. Auch können die Polymerisate zum Verfärben neigen.

Deshalb ist man für solche Anwendungszwecke bestrebt, die Verunreinigungen aus der Roh-(Meth)acrylsäure weitgehend abzutrennen und Roh-(Meth)acrylsäure in Rein-(Meth)acrylsäure überzuführen. Dies erfolgt in der Regel auf destillativem Weg, z.B. durch zwei sich anschließende Rektifikationsstufen zur Entfernung leichter und schwerer als die (Meth)acrylsäure siedender Verunreinigungen (vgl. z.B. EP-B 102 642).

Problematisch ist jedoch, daß wenigstens ein Teil der aldehydischen Verunreinigungen in seinem physikalischen Verhalten der (Meth)acrylsäure so ähnlich ist, daß eine ausschließlich rektifikative Entfernung derselben nur unter Anwendung einer unwirtschaftlichen Anzahl von Trennböden und/oder eines unwirtschaftlichen Rücklaufverhältnisses möglich ist.

Aus der DE-B 22 07 184 und der GB-PS 1 346 737 ist daher ein Verfahren zur Reinigung von Roh-Acrylsäure bekannt, das dadurch gekennzeichnet ist, daß man der Roh-Acrylsäure wenigstens ein primäres Amin wie z.B. Hydrazin, Phenylhydrazin, Anilin, Monoethanolamin, Ethylendiamin und Glycin zugibt und aus dem Gemisch die Acrylsäure destillativ abtrennt.

Die primären Amine binden offensichtlich die als Verunreinigungen enthaltenen Aldehyde in hohem Ausmaß, so daß bereits eine sich anschließende einfache destillative Trennstufe bezüglich der aldehydischen Verunreinigungen eine hohe Trennwirkung erzielt.

Die EP-A 270 999 empfiehlt in entsprechender Weise Roh-(Meth)acrylsäure vor der destillativen Aufarbeitung mit Aminoguanidin und/oder dessen Salzen in Mengen von 1 bis 3 Mol pro Mol enthaltenem Aldehyd zu versetzen.

Aus der DE-A 4 201 697 ist bekannt, Roh-(Meth)acrylsäure vor der destillativen Aufarbeitung mit einer Arylsulfonsäure zu versetzen. In ähnlicher Weise umfaßt die DE-B 2 207 184 auch noch die Empfehlung vorab einer destillativen Aufarbeitung Schwefelsäure zuzugeben. Die JP-A 117 716/75 empfiehlt die Kombination Phenothiazin/Sulfonsäure als Polymerisationsinhibitor für Acrylsäure.

Nachteilig an diesen Verfahrensweisen des Standes der Technik ist jedoch, daß sich während der Destillation die Destillationsvorrichtungen (insbesondere die Verdampferoberfläche) relativ rasch mit Belag bedecken, der durch das Beisein der vorgenannten Additive bedingt wird, da er bei einer destillativen Aufarbeitung der Roh-(Meth)acrylsäure in Abwesenheit dieser Zusätze nicht auftritt (allerdings bedingt eine solche zusatzfreie destillative Aufarbeitung nur eine geringe Trennwirkung bezüglich der in der Roh-(Meth)acrylsäure enthaltenen aldehydischen Verunreinigungen).

Offensichtlich sind an der Belagsbildung unmittelbare Umsetzungsprodukte der Zusätze mit den aldehydischen Verunreinigungen und/oder im Rahmen der destillativen Aufarbeitung aus diesen entstehende Folgeprodukte beteiligt.

Aufgabe der vorliegenden Erfindung war daher ein Verfahren zum Abtrennen von Aldehyden aus Roh-(Meth)acrylsäure, die nach dem Verfahren der katalytischen Gasphasenoxidation erzeugt wurde, bei dem man die Roh-(Meth)acrylsäure mit einem primären Amin und/oder dessen Salzen versetzt und aus dem Gemisch die (Meth)acrylsäure destillativ abtrennt, bei dem die beschriebene Belagsbildung gemindert wird.

Demgemäß wurde ein Verfahren zum Abtrennen von Aldehyden aus Roh-(Meth)acrylsäure, die nach dem Verfahren der katalytischen Gasphasenoxidation erzeugt wurde, bei dem man die Roh-(Meth)acrylsäure mit einem primären Amin und/oder dessen Salzen versetzt und aus dem Gemisch die (Meth)acrylsäure destillativ abtrennt, gefunden, das dadurch gekennzeichnet ist, daß man der Roh-(Meth)acrylsäure vor der destillativen Behandlung zusätzlich zum zugesetzen primären Amin und/oder dessen Salzen wenigstens eine organische Sulfonsäure und/oder deren Salze (insbesondere die Alkalimetallsalze) zusetzt. Von Vorteil ist es, wenn die organische Sulfonsäure so beschaffen ist, daß sie bei Zusatz zu Wasser bei 25°C die Oberflächenspannung desselben reduziert. In der Regel wird man pro Mol zugesetzter primärer Aminverbindung wenigstens 0,1 Mol, üblicherweise aber nicht mehr als 5 Mol wenigstens einer organischen Sulfonsäure zusetzen, da oberhalb dieser Zusatzmenge keine verbesserte Wirkung erzielt wird. Vorzugsweise werden pro Mol zugesetzter primärer Aminverbindung 0,5 bis 2 Mol wenigstens einer organischen Sulfonsäure zugesetzt.

Bevorzugt erfolgt der Zusatz der wenigstens einen organischen Sulfonsäure erst kurz vor der destillativen Aufarbeitung. D.h. man setzt der Roh-(Meth)acrylsäure zweckmäßigerweise zunächst ausschließlich die primäre Aminverbindung zu, überläßt das Gemisch mit Vorteil einige Zeit sich selbst (die Reaktionsdauer hängt von der Temperatur ab; bei Temperaturen von 20 bis 100°C liegt sie zwischen wenigen Minuten und einigen Stunden; anwendungstechnisch vorteilhaft erfolgt das sich selbst überlassen bei Raumtemperatur), setzt danach die wenigstens eine Arylsulfonsäureverbindung zu und arbeitet anschließend destillativ auf. Wahlweise kann die Zugabe der organischen Sulfonsäure auch vor oder zeitgleich mit der Zugabe der primären Aminverbindung erfolgen. Ferner kann der Zusatz der organischen Sulfonsäure auch nur auf den Böden der Destillationskolonne, am Kolonnenkopf oder über den Rücklauf zur Kolonne erfolgen. Der Zusatz der organischen Sulfonsäure ist auch dann erfolgreich, wenn bereits Belagsbildung erfolgt ist.

Geeignete organische Sulfonsäuren sind z.B. Alkylsulfonsäuren der allgemeinen Formel

R¹-SO₃H,

in der R¹ für C₁- bis C₂₀-Alkyl steht. Ferner eignen sich die Salze dieser Alkylsulfonsäuren, insbesondere die Alkalimetallsalze. Ein bevorzugter Vertreter ist die Methansulfonsäure und deren Salze.

Ferner eigenen sich Arylsulfonsäuren und deren Salze. Geeignete Arylsulfonsäureverbindungen sind Toluolsulfonsäuren wie p-Toluolsulfonsäure, Benzolsulfonsäure, Phenolsulfonsäuren, Xylolsulfonsäuren, Dibutylnaphthalinsulfonsäuren und deren Salze, insbesondere deren Alkalimetallsalze. Ganz besonders eignen sich jedoch Alkylarylsulfonsäuren und deren Salze, unter denen wiederum jene bevorzugt sind, die nur einen Alkylsubstituenten aufweisen. Aryl steht vorzugsweise für ein Benzol- oder Naphtalin-Ringsystem.

Mit Vorteil weist der Alkylrest 5 bis 16 C-Atome auf.

Von spezieller Eignung sind die Alkylbenzolsulfonsäuren und deren Salze, d.h. Verbindungen der allgemeinen Formel 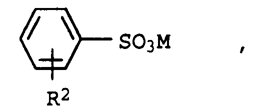 in der M für Wasserstoff oder ein anderes Kation, z.B. ein Alkalimetallion, und R² für C₅- bis C₁₆-Alkyl steht.

Vorzugsweise steht R² für C₈- bis C₁₂-Alkyl.

Unter den Alkylbenzolsulfonsäureverbindungen der oben angeführten allgemeinen Formel nehmen wiederum die Dodecylbenzolsulfonsäuren und deren Salze eine herausragende Position ein. Dies gilt insbesondere für Dodecylbenzolsulfonsäureverbindungen der allgemeinen Formel 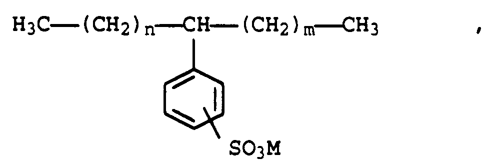 in der n, m ganze Zahlen bedeuten, deren Summe 9 ergeben muß. Ganz generell wird die Arylsulfonsäureverbindung vorzugsweise in ihrer vollsauren Form zugesetzt. D.h. die Arylsulfonsäure ist das bevorzugte Additiv. Dies gilt ebenso für die Alkylsulfonsäureverbindungen sowie für Aralkylsulfonsäureverbindungen, die sich ebenfalls als erfindungsgemäß zuzusetzende organische Sulfonsäureverbindung eignen.

Geeignete zuzusetzende primäre Amine und/oder deren Salze sind alle diejenigen, deren Zusatz für sich bereits der Stand der Technik empfiehlt. Als solche seien beispielhaft genannt (aufgelistet wird der Einfachheit halber lediglich die Aminform; die Salze ergeben sich dazu entsprechend) Hydrazin und dessen Derivate wie z.B. Guanylhydrazin (Aminoguanidin) u. Phenylhydrazin, aromatische Amine, die vorzugsweise bis zu 12 C-Atome aufweisen, wie z.B. Anilin, o-, m-, p-Toluidin und o-, m-, p-Anilin, Aminocarbonsäuren wie z.B. Glycin, Aminoalkohole wie z.B. Ethanolamin (2-Aminoethanol), aber auch kettenförmige, verzweigte und cyclische aliphatische Amine, die 1 bis 12 C-Atome aufweisen wie z.B. Methylamin. Selbstverständlich kommen auch mehrwertige primäre Amine in Betracht. D.h. es eignen sich auch Verbindungen, die mehr als eine, beispielsweise 2, 3 oder 4 -NH₂ Gruppen aufweisen. Beispielhaft genannt seien 1,2-Diaminoethan, Putrescin (Tetramethylendiamin), Cadaverin (Pentamethylendiamin). Selbstverständlich eignen sich auch Verbindungen, die sowohl eine -NH₂- als auch eine -SO₃H Gruppe aufweisen, wie z.B. Sulfanilsäure (4-Aminobenzolsulfonsäure).

Geeignete Salze der zuzusetzenden primären Amine sind insbesondere deren Hydrogencarbonat, Nitrat, Sulfat oder Chlorid. Beispielhaft genannt sei das Aminoguanidin-Hydrogencarbonat, wobei das Aminoguanidin-Hydrogencarbonat die vorzugsweise zugesetzte Aminoguanidinverbindung ist. MIt besonderem Vorteil besteht die zugesetzte Kombination ausschließlich aus Aminoguanidin-Hydrogencarbonat und Dodecylbenzolsulfonsäure.

Selbstverständlich läßt sich das erfindungsgemäße Verfahren auch zur Abtrennung von Aldehyden aus (Meth)acrylsäure anwenden, bei der es sich nicht um Roh-(Meth)acrylsäure handelt, d.h. die auf anderem Weg als durch gasphasenkatalytische Oxidation von C₃-, C₄-Verbindungen erhalten worden und somit ursächlich aus anderen Gründen mit aldehydischen Verunreinigungen behaftet ist.

Vorzugsweise wird die destillative Aufarbeitung der erfindungsgemäß modifizierten Roh-(Meth)acrylsäure bei reduziertem Druck, zweckmäßigerweise ≤ 100 mbar, in der Regel 10 bis 100 mbar, vorgenommen. Entsprechend liegt die zugehörige Siedetemperatur üblicherweise im Bereich von 70 bis 105°C.

Von besonderer Bedeutung ist das erfindungsgemäße Verfahren im Fall von Roh-Methacrylsäure, deren gasphasenkatalytisch oxidative Herstellung ausgehend von Methacrolein erfolgt, insbesondere dann, wenn das Methacrolein durch gasphasenkatalytische Oxidation von tert.-Butanol, iso-Butan oder iso-Buten oder durch Umsetzung von Formaldehyd mit Propionaldehyd gemäß EP-B 92 097 oder EP-B 58 927 erzeugt wird, und dieses insbesondere dann, wenn die gasphasenkatalytische Oxidation des tert. Butanols, iso-Butans oder iso-Butens unter Verwendung einer katalytisch aktiven Masse der allgemeinen Formel I

Mo₁₂BiₐFe_{b}X¹_{c}X²_{d}X³ₑX⁴_{g}Oₙ (I),

in der die Variablen folgende Bedeutung haben:
- X¹: Nickel und/oder Kobalt,
- X²: Thallium, ein Alkalimetall und/oder ein Erdalkalimetall,
- X³: Phoshor, Arsen, Bor, Antimon, Zinn, Cer, Blei, Niob und/oder Wolfram,
- X⁴: Silicium, Aluminium, Titan und/oder Zirkonium,
- a: 0,5 bis 5,0
- b: 0,01 bis 3,0
- c: 3,0 bis 10,0
- d: 0,02 bis 2,0
- e: 0 bis 5,0
- g: 0 bis 10 und
- n: eine Zahl, die durch die Wertigkeit und Häufigkeit der von Sauerstoff verschiedenen Elemente in I bestimmt wird,
bei Temperaturen von 300 bis 400°C und, vom speziellen Temperaturverlauf abgesehen, ansonsten gemäß den Bedingungen der älteren deutschen Anmeldung P 40 23 239.5 (O.Z. 0050/41774) erfolgt und das anfallende Methacrolein ohne Zwischenreinigung zur Weiteroxidation verwendet wird. Ferner bewährt sich das erfindungsgemäße Verfahren besonders dann, wenn die gasphasenkatalytische Oxidation des Methacroleins, vom speziellen Temperaturverlauf abgesehen, gemäß den Ausführungen der älteren deutschen Anmeldung P 41 32 263.0 (O.Z. 0050/42719) bei Temperaturen von 200 bis 350°C bzw. gemäß der älteren deutschen Anmeldung P 41 32 684.9 (O.Z. 0050/42725) bei Temperaturen von 250 bis 400°C erfolgt.

Selbstverständlich erfolgt das erfindungsgemäße Verfahren in Gegenwart von Polymerisationsinhibitoren wie Luft, Hydrochinon, Hydrochinonmonoethylether, Paranitrosophenol, Paramethoxyphenol oder Phenothiazin.

Üblicherweise werden sie, bezogen auf die Roh(Meth)acrylsäure, in Mengen von 50 bis 1000 ppm eingesetzt.

Besonders vorteilhaft hat sich das erfindungsgemäße Verfahren in Gegenwart von aromatischen Hydroxyverbindungen wie Hydrochinon und Hydrochinonmonomethylether als Polymerisationsinhibitoren erwiesen.

Das erfindungsgemäße Verfahren ist dadurch ausgezeichnet, daß es einerseits eine besonders effektive destillative Abtrennung aldehydischer Verunreinigungen aus Roh-(Meth)acrylsäure bedingt und andererseits eine damit einhergehende Belagsbildung weitgehend unterdrückt. Das erfindungsgemäße Verfahren eignet sich daher insbesondere für eine kontinuierliche Ausführungsweise, wie sie in der DE-A 42 01 697 beschrieben ist.

### Beispiele

### Beispiel 1

Gemäß Beispiel 1 der EP-B 58 927 wurde durch Kondensation von Formaldehyd und Propionaldehyd und anschließende azeotrope Destillation Methacrolein hergestellt. Aus diesem wurde gemäß dem Ausführungsbeispiel der EP-B 297 445 (katalytische Gasphasenoxidation) eine wäßrige Methacrylsäurelösung gewonnen. Dies wurde mit der für die Oxidation eingesetzten Syntheseluft (Frischluft) bei 50 bis 70°C gestrippt (Frischluftmenge siehe Tabelle 1 der EP-A 297 445), danach gemäß Beispiel 6 der Patentschrift 54 354 der Deutschen Demokratischen Republik mit Isooktansäure extrahiert und das dabei anfallende Methacrylsäure/Isooktansäure-Gemisch gemäß Beispiel 10 derselben Schrift destillativ aufgetrennt. Die so erhaltene Roh-Methacrylsäure wurde mit 944 Gew.-ppm Aminoguanidin-Hydrogencarbonat (bezogen auf die Roh-säure) versetzt und das resultierende Gemisch bei Raumtemperatur ca. 6 h unter leichtem Rühren sich selbst überlassen.

Anschließend wurde die so behandelte Roh-Methacrylsäure kontinuierlich (0,5 l/h) einer Rektifikationskolonne zur über Kopf erfolgenden Abtrennung von niedriger als Methacrylsäure siedenden Verunreinigungen zugeführt. Der Sumpfaustrag dieser Kolonne wurde kontinuierlich in eine Destillationsblase überführt und die Methacrylsäure über Kopf in hoher Reinheit abdestilliert (Blasentemperatur: 105°C, Druck: 100 mbar, Aldehyd-Reinheit der resultierenden Methacrylsäure: < 1 ppm CO (gerechnet als Methacrolein)). Bei der verwendeten Rektifikationskolonne handelte es sich um eine isolierte, doppelwandig verspiegelte Füllkörperkolonne (Durchmesser: 50 mm, Länge: 1 m, 5 mm Netzdrahtwendeln, Sumpftemperatur: 105°C, Druck: 100 mbar).

Bereits nach einer kontinuierlichen Betriebsdauer von 2 h zeigte sich im Sumpf der Rektifikationskolonne und in der Destillationsblase starker Feststoffniederschlag (rehbrauner Schlamm), der einerseits zu Belagsbildung, welche die Wärmeübertragung behindert, und andererseits zum Verstopfen von Leitungen und Pumpen führte, so daß die Anlage nach 3 h abgestellt werden mußte.

Das Ausführungsbeispiel wurde unter gleichen Bedingungen wiederholt, jedoch wurde vor der destillativen Aufarbeitung zusätzlich eine zur zugesetzten Menge an Aminoguanidin-Hydrogencarbonat äquimolare Menge an Dodecylbenzolsulfonsäure zugesetzt.

Nach einer kontinuierlichen Betriebsdauer von 48 h war noch keine Feststoffabscheidung im Sumpf der Rektifikationskolonne und in der Destillationsbase festzustellen. Die Aldehyd-Reinheit der resultierenden Methacrylsäure betrug ebenfalls < 1 ppm CO (gerechnet als Methacrolein).

In beiden Fällen wurde in Anwesenheit von 200 ppm (bezogen auf das Gewicht der Rohsäuren) Hydrochinonmonomethylether als Polymerisationsinhibitor gearbeitet.

### Beispiel 2

In eine kontinuierlich zu betreibende Rektifikationskolonne aus Glas, deren Verdampfer ein Konvektionsumlaufverdampfer aus Glas war, der mittels einer metallischen, elektrisch beheizbaren Kerze beheizt wurde, wurden über den Verdampfer kontinuierlich 140 ml/h einer Roh-Acrylsäure zugeführt, die durch katalytische Gasphasenoxidation von Acrolein gemäß Beispiel B1 der DE-A 43 02 991 und anschließende Aufarbeitung der Reaktionsgase gemäß Beispiel B1 der DE-A 21 36 396 erhalten worden war. Die gleiche Menge an Roh-säure wurde dem Verdampfer kontinuierlich entnommen. Die verdampfte Rohsäure wurde oberhalb der Rektifikationskolonne kondensiert und als Rücklauf quantitativ wieder auf den Kolonnenkopf gegeben. Eine Stabilisierung der Kolonne erfolgte mittels Phenothiazin über den Kolonnenkopf. Der Roh-Acrylsäure waren vor der Zufuhr in den Verdampfer 1100 ppm ihres Gewichtes an Aminoguanidinhydrogencarbonat zugesetzt worden. Während der Rektifikation trat auf der Heizkerze Belagsbildung auf, deren Menge nach einer Betriebsdauer von 40 h ausgewogen wurde. Das Ergebnis zeigt die nachfolgende Tabelle. Sie zeigt ferner die Ergebnisse die erhalten wurden, wenn der Roh-Acrylsäure unmittelbar vor ihrem Zulauf in den Verdampfer pro Mol enthaltener Aminoguanidinverbindung 1,5 mol verschiedener organischer Sulfonsäuren zugesetzt wurden.

**Tabelle**

| Zugesetzte Sulfonsäure | Belagsmenge (mg) |
|---|---|
| - | 400 |
| p-Toluolsulfonsäure | 50 |
| Dodecylbenzolsulfonsäure | 20 |
| Methansulfonsäure | 10 |
| Xylolsulfonsäure | 50 |
| Dibutylnaphthalinsulfonsäure | 70 |

### Beispiel 3

Wie Beispiel 2, anstelle von 1100 ppm Aminoguanidinhydrogencarbonat wurden jedoch 450 ppm Hydrazinmonohydrat zugesetzt. Nach einer Betriebsdauer von 5 h betrug die Belagsbildung 2200 mg. Wurden der Roh-Acrylsäure unmittelbar vor ihrem Zulauf in den Verdampfer pro Mol zugesetzten Hydrazins zusätzlich 1,5 mol Dodecylbenzolsulfonsäure zugesetzt, lag die gebildete Belagsmenge auch nach 40 Betriebsstunden noch unterhalb 10 mg.

## Patentansprüche

1. Verfahren zur Reinigung einer mit Aldehyden verunreinigten (Meth)acrylsäure, bei dem man die (Meth)acrylsäure mit einem primären Amin und/oder dessen Salzen versetzt und aus dem Gemisch die (Meth)acrylsäure destillativ abtrennt, dadurch gekennzeichnet, daß man der mit Aldehyden verunreinigten (Meth)acrylsäure vor der destillativen Behandlung zusätzlich zum zugesetzten primären Amin und/oder dessen Salzen wenigstens eine organische Sulfonsäure und/oder eines deren Salze zusetzt.

2. Verfahren nach Anspruch 1, dadurch gekennzeichnet, daß man pro Mol aldehydischer Verunreinigungen bis zu 5 Mol an primärem Amin und/oder dessen Salzen zusetzt.

3. Verfahren nach Anspruch 1 oder 2, dadurch gekennzeichnet, daß man pro Mol zugesetzter primärer Aminverbindung bis zu 5 Mol wenigstens einer organischen Sulfonsäure und/oder eines deren Salze zusetzt.

4. Verfahren nach Anspruch 1 bis 3, dadurch gekennzeichnet, daß man pro Mol aldehydischer Verunreinigungen 1 bis 3 Mol an primärem Amin und/oder dessen Salzen, sowie pro Mol zugesetzter primärer Aminverbindung 0,5 bis 2 Mol wenigstens einer organischen Sulfonsäure und/oder eines deren Salze zusetzt.

5. Verfahren nach Anspruch 1 bis 4, dadurch gekennzeichnet, daß die zugesetzte primäre Aminverbindung Hydrazin und/oder ein Hydrazinderivat umfaßt.

6. Verfahren nach Anspruch 1 bis 5, dadurch gekennzeichnet, daß die zugesetzte primäre Aminverbindung Aminoguanidin und/oder dessen Salze umfaßt.

7. Verfahren nach Anspruch 6, dadurch gekennzeichnet, daß die zugesetzte primäre Aminverbindung Aminoguanidin-Hydrogencarbonat umfaßt.

8. Verfahren nach Anspruch 1 bis 7, dadurch gekennzeichnet, daß die zugesetzte Sulfonsäureverbindung Dodecylbenzolsulfonsäure umfaßt.

9. Verfahren nach Anspruch 1 bis 8, dadurch gekennzeichnet, daß die mit Aldehyden verunreinigte (Meth)acrylsäure eine Roh-(Meth)acrylsäure ist, die nach dem Verfahren der katalytischen Gasphasenoxidation erzeugt worden ist.

10. Verfahren nach Anspruch 1 bis 9, dadurch gekennzeichnet, daß es kontinuierlich ausgeführt wird.

## Claims

1. A process for purifying a (meth)acrylic acid contaminated with aldehydes, in which a primary amine or a salt thereof is added to the (meth)acrylic acid and the (meth)acrylic acid is separated from the mixture by distillation, wherein, in addition to the added primary amine or its salts, at least one organic sulfonic acid or one of its salts is added to the (meth)acrylic acid contaminated with aldehydes, before the treatment by distillation.

2. A process as claimed in claim 1, wherein up to 5 mol of primary amine or a salt thereof are added per mole of aldehydic impurities.

3. A process as claimed in claim 1 or 2, wherein up to 5 mol of at least one organic sulfonic acid or of one of its salts are added per mole of added primary amine compound.

4. A process as claimed in any of claims 1 to 3, wherein from 1 to 3 mol of primary amine or of a salt thereof are added per mole of aldehydic impurities, and from 0.5 to 2 mol of at least one organic sulfonic acid or of a salt thereof are added per mole of added primary amine compound.

5. A process as claimed in any of claims 1 to 4, wherein the added primary amine compound comprises hydrazine or a hydrazine derivative.

6. A process as claimed in any of claims 1 to 5, wherein the added primary amine compound comprises aminoguanidine or a salt thereof.

7. A process as claimed in claim 6, wherein the added primary amine compound comprises aminoguanidine bicarbonate.

8. A process as claimed in any of claims 1 to 7, wherein the added sulfonic acid compound comprises dodecylbenzenesulfonic acid.

9. A process as claimed in any of claims 1 to 8, wherein the (meth)acrylic acid contaminated with aldehydes is a crude (meth)acrylic acid which has been produced by the catalytic gas-phase oxidation method.

10. A process as claimed in any of claims 1 to 9, which is carried out continuously.

## Revendications

1. Procédé de purification d'acide (méth)acrylique pollué par des aldéhydes dans lequel on mélange l'acide (méth)acrylique avec une amine primaire et/ou ses sels et on extrait par distillation l'acide (méth)acrylique du mélange, caractérisé en ce que l'on ajoute à l'acide (méth)acrylique pollué par des aldéhydes au moins un acide sulfonique organique et/ou l'un de ses sels, avant le traitement par distillation, en plus de l'amine primaire et/ou de ses sels déjà ajoutés.

2. Procédé selon la revendication 1, caractérisé en ce que l'on ajoute jusqu'à 5 moles d'amine primaire et/ou de ses sels par mole d'impuretés aldéhydiques.

3. Procédé selon la revendication 1 ou 2, caractérisé en ce que l'on ajoute jusqu'à 5 moles d'au moins un acide sulfonique organique et/ou d'un de ses sels par mole de composé amine primaire ajouté.

4. Procédé selon l'une quelconque des revendication 1 à 3, caractérisé en ce que l'on ajoute 1 à 3 moles d'amine primaire et/ou de ses sels par mole d'impuretés aldéhydiques et 0,5 à 2 moles d'au moins un acide sulfonique organique et/ou d'un de ses sels par mole de composé amine primaire ajouté.

5. Procédé selon l'une quelconque des revendications 1 à 4, caractérisé en ce que le composé amine primaire ajouté comprend l'hydrazine et/ou un dérivé de l'hydrazine.

6. Procédé selon l'une quelconque des revendications 1 à 5, caractérisé en ce que le composé amine primaire ajouté comprend l'aminoguanidine et/ou ses sels.

7. Procédé selon la revendication 6, caractérisé en ce que le composé amine primaire ajouté comprend l'hydrogénocarbonate d'aminoguanidine.

8. Procédé selon l'une quelconque des revendications 1 à 7, caractérisé en ce que le composé acide sulfonique ajouté comprend l'acide dodécylbenzènesulfonique.

9. Procédé selon l'une quelconque des revendications 1 à 8, caractérisé en ce que l'acide (méth)acrylique pollué par des aldéhydes est un acide (méth)acrylique brut, obtenu par le procédé d'oxydation catalytique en phase gazeuse.

10. Procédé selon l'une quelconque des revendications 1 à 9, caractérisé en ce qu'il est mené en continu.
